# EUROPÄISCHE PATENTSCHRIFT

(11) **EP 2 448 619 B1**
(45) Veröffentlichungstag und Bekanntmachung des Hinweises auf die Patenterteilung: **03.05.2017**
(21) Anmeldenummer: 10725624.0
(22) Anmeldetag: 17.06.2010
(51) Int. Cl.: A61M 5/30, A61M 5/32, A61M 5/42

(54) **ZYLINDER-KOLBEN-EINHEIT EINES EINWEGINJEKTORS MIT ERHÖHTER BETRIEBSSICHERHEIT**
CYLINDER-PISTON UNIT OF A DISPOSABLE INJECTOR HAVING INCREASED OPERATIONAL SAFETY
UNITÉ CYLINDRE-PISTON D'UN INJECTEUR JETABLE À SÉCURITÉ DE FONCTIONNEMENT ACCRUE

(30) Priorität: 30.06.2009 DE 102009031303
(43) Veröffentlichungstag der Anmeldung: 09.05.2012
(73) Patentinhaber: LTS LOHMANN Therapie-Systeme AG, 56626 Andernach (DE)
(72) Erfinder: MATUSCH, Rudolf, 35041 Marburg (DE)
(74) Vertreter: Thämer, Wolfgang
(86) Internationale Anmeldenummer: PCT/EP2010/003674
(87) Internationale Veröffentlichungsnummer: WO 2011/000484

(56) Entgegenhaltungen:
- EP-A2- 1 354 609
- WO-A1-2007/054233
- WO-A1-2007/088112
- WO-A1-2009/111794
- WO-A2-2004/069301
- WO-A2-2008/083209
- DE-C- 957 598
- US-A- 5 503 627

## Beschreibung

Die Erfindung betrifft eine Zylinder-Kolben-Einheit eines Einmalinjektors mit mindestens einem, den Zylinderinnenraum mit einer stirnseitigen Mündung verbindenden Durchbruch, wobei eine Stirnseite der Zylinder-Kolben-Einheit einen die Mündung umgreifenden Einpressbereich und einen den Einpressbereich umgebenden Anpressbereich aufweist, wobei der Einpressbereich einen inneren, die Mündung begrenzenden Steg und einen äußeren Steg umfasst, wobei die Stege eine den inneren Steg umgebende Ausnehmung begrenzen, deren parallel zur Mittelachse der Zylinder-Kolben-Einheit gemessene Tiefe mindestens ein Viertel ihrer Breite in einer normal hierzu orientierten Ebene beträgt und wobei die in eine Normalenebene zur Mittelachse projizierte Fläche des Anpressbereichs mindestens so groß ist wie die in dieselbe Ebene projizierte Fläche des Einpressbereichs.

Die EP 1 354 609 A2 offenbart einen Mehrfachinjektor mit einer Zylinder-Kolben-Einheit. Die Zylinder-Kolben-Einheit hat einen zentralen zylinderförmigen Zapfen mit einem Längskanal. Bei einer Injektion drückt dieser Zapfen in die Haut, während ein äußerer Ring ist auf der Haut aufliegt. Ein weiterer, den zylindrischen Zapfen umgebender Versteifungsring hat bei der Injektion keine Funktion und kommt nicht mit der Haut des Patienten in Kontakt. Die punktuelle Belastung durch den zylindrischen Zapfen kann zu Einrissen der Haut führen. Außerdem besteht die Gefahr des Wegrutschens des Injektors.

Zylinder-Kolben-Einheiten sind außerdem z.B. aus US 5 503 627 A, WO 2009/111794 A1, WO 2007/054233 A1, WO 2004/069301 A2 oder WO 2008/083209 A2 bekannt.

Aus der DE 957 598 B ist eine weitere Zylinder-Kolben-Einheit bekannt. Um die Bildung eines Einrisses statt eines Penetrationskanals zu verhindern, wird gegen das seitliche Verrutschen ein eingekerbter Friktionskranz ausgebildet.

Der vorliegenden Erfindung liegt daher die Problemstellung zugrunde, die Betriebssicherheit einer Zylinder-Kolben-Einheit eines Einmalinjektors weiter zu erhöhen und einen sogenannten "wet shot" zu verhindern.

Diese Problemstellung wird mit den Merkmalen des Hauptanspruches gelöst. Dazu weist der innere Steg eine konkav ausgebildete Außenflanke und eine Innenflanke auf, die in einer Kante einen Winkel von bis zu 45 Grad einschließen. Der äußere Steg ist mittels zweier konkaver Flanken begrenzt, die in einer Kante einen Winkel von bis zu 45 Grad einschließen. Die Kanten liegen entweder in einer Ebene normal zur Mittelachse oder spannen eine kegelförmige Fläche auf, deren Spitzenwinkel zwischen 165 Grad und 180 Grad beträgt und deren Kegelspitze außerhalb der Zylinder-Kolben-Einheit liegt, Außerdem ist der Anpressbereich zumindest während einer Injektion entgegen der Injektionsrichtung zu der Stirnfläche des Einpressbereichs versetzt.

Weitere Einzelheiten der Erfindung ergeben sich aus den Unteransprüchen und der nachfolgenden Beschreibung schematisch dargestellter Ausführungsformen.
- Figur 1:: Zylinder-Kolben-Einheit mit Verschlusskappe;
- Figur 2:: Detail von Figur 1;
- Figur 3:: Draufsicht der Stirnseite;
- Figur 4:: Auf die Haut aufgesetzte Zylinder-Kolben-Einheit;
- Figur 5:: Stirnseite mit im Sechseck angeordneten äußerem und inneren Steg;
- Figur 6:: Zylinder-Kolben-Einheit mit mehreren Durchbrüchen;
- Figur 7:: Draufsicht auf Figur 6;
- Figur 8:: Zylinder-Kolben-Einheit mit zwei Abdichtbereichen;
- Figur 9:: Detail des Einpressbereichs;
- Figur 10:: Zylinder-Kolben-Einheit mit axial zueinander verschiebbaren Einpress- und Anpressbereich;
- Figur 11:: Figur 10 bei einer Injektion.

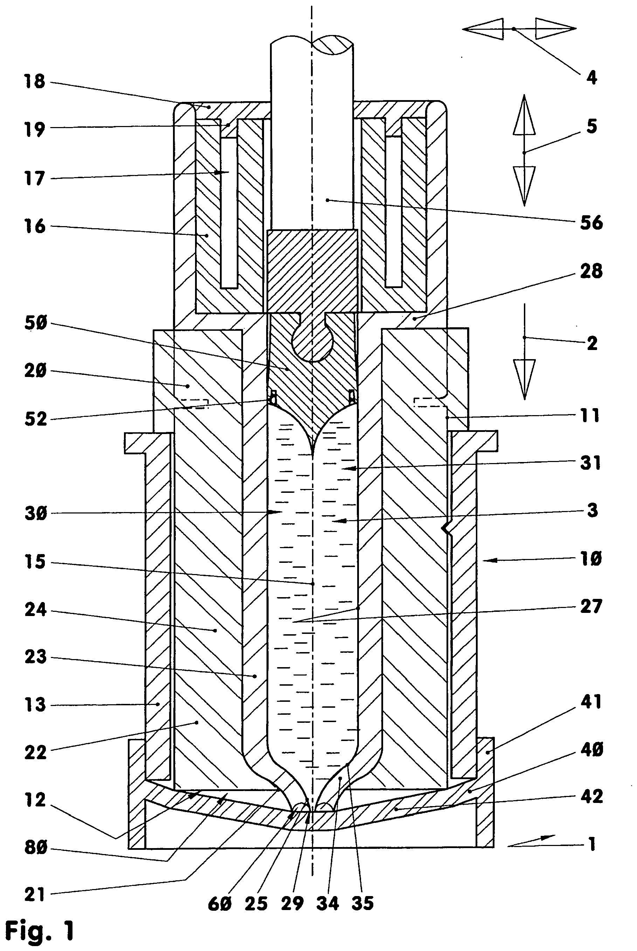

Die Figuren 1 - 4 zeigen eine Zylinder-Kolben-Einheit 10 eines Injektors, z.B. eines Einweginjektors. Derartige Zylinder-Kolben-Einheiten 10 werden beispielsweise zur Lagerung und zur Ausbringung von Wirkstoffen in nadellosen Injektoren oder in Injektoren mit integrierter Injektionsnadel eingesetzt.

Die Zylinder-Kolben-Einheit 10 umfasst einen Zylinder 20 und einen Kolben 50. Beim Einsatz der Zylinder-Kolben-Einheit 10 in einem Injektor schließen der Zylinder 20 und der Kolben 50 in einem Verdrängungsraum 30 ein subkutan, intradermal oder intramuskulär zu verabreichendes Präparat 3 oder ein flüssiges Trägermaterial, z.B. destilliertes Wasser oder physiologische Kochsalzlösung, ein.

Die Zylinder-Kolben-Einheit 10 ist beispielsweise für den einmaligen Gebrauch vorgesehen. Sie dient der Verabreichung eines Arzneinennvolumen von z.B. 0,1 bis 2 ml. Ggf. kann auch ein Arzneinennvolumen von 3 ml realisiert werden.

Der Zylinder 20 hat in erster Näherung die Form des Spitzenzylinders einer genormten Einmalspritze. Beispielsweise hat er einen Innendurchmesser von 5,6 Millimetern und einen Außendurchmesser von 16,6 Millimetern. Am vorderen Ende 21 verbindet ein Durchbruch 25 den Zylinderinnenraum 31 mit der Umgebung 1. Dieser Durchbruch 25 umfasst ein düsenartiges Austrittselement 26 mit einem Durchmesser von z.B. 0,2 Millimetern.

Der Ausströmtrichter 35 verjüngt sich zwischen dem Zylinderboden 34 und der Düsenbohrung 26 zur besseren Strömungsführung nicht linear. Der Übergang zwischen dem Ausströmtrichter 35 und der Düsenbohrung 26 ist beispielsweise stetig. Die Düsenbohrung 26, deren Durchmesser z.B. zwischen 0,1 und 0,5 Millimetern liegt, ist zwei- bis viermal so lang wie ihr Durchmesser.

Gegebenenfalls kann der Zylinder 20 mehrere Durchbrüche 25 umfassen, die z.B. auf einem gemeinsamen Teilkreis um die Mittellinie 15 oder um einen zentralen Durchbruch 25 angeordnet sind.

Der z.B. topfartig ausgebildete Zylinder 20 hat im Ausführungsbeispiel zumindest bereichsweise eine mehrschichtige Wand 22. Die an den Zylinderinnenraum 31 angrenzende Innenwandschicht 23, die auch den Durchbruch 25 umgreift, besteht z.B. aus einem amorphen, transparenten Thermoplast, z.B. aus einem oder aus mehreren Copolymeren auf der Basis von Cycloolefinen und Ethylenen oder α-Olefinen (COC) und hat eine Wandstärke von 1,5 Millimetern. An diese Innenwandschicht 23 ist z.B. im Zwei-Komponenten-Spritzgussverfahren eine hülsenförmige Außenwandschicht 24 angeformt. Diese Außenwandschicht 24 ist z.B. aus Polycarbonat (PC) hergestellt und hat im Ausführungsbeispiel eine Wandstärke von vier Millimetern.

Die Zylinderwand 22 kann einschichtig ausgebildet sein, sie ist dann beispielsweise aus COC hergestellt. Sie kann auch - bei ein- oder mehrschichtigem Aufbau - eine Verstärkung, z.B. ein in die Wand 22 integriertes Drahtgeflecht aufweisen.

Der Kolben 50 weist auf seiner dem Verdrängungsraum 30 zugewandten Stirnseite eine umlaufende, schürzenartige Dichtlippe 52 auf. Diese Dichtlippe 52 presst vorgespannt gegen die Zylinderinnenwandung 27. Mit der beim Kolbenhub zunehmenden Gegenkraft steigt die Anpresskraft dieser Dichtlippe 52 an die Zylinderinnenwandung 27. Der Kolben 50 ist beispielsweise aus Teflon^{®} hergestellt.

Am Kolben 50 ist eine Kolbenstange 56 befestigt. Diese hat im Ausführungsbeispiel einen Durchmesser von fünf Millimetern und ist aus COC hergestellt.

Der Zylinder 20 weist an seinem dem Durchbruch 25 abgewandten Ende eine abgestufte Querschnittserweiterung 28 auf. In dieser Querschnittserweiterung 28 sitzt ein zweiteiliger Sterilitätsverschluss 16, 18. Ein erstes, U-förmiges Profilteil 16 liegt an der Zylinderinnenwandung 27 und an der Kolbenstange 56 an. In der U-förmigen Öffnung 17 sitzt ein Dichtring 18 mit einem Steg 19. Dieser presst das U-förmige Profilteil 16 an die beiden genannten Bauteile 27, 56.

Zur Verbindung mit dem Antrieb hat die Zylinder-Kolben-Einheit 10 z.B. ein Gewinde 11, einen Bajonettanschluss, einen Aufnahmeschlitz, etc.

Die Stirnseite 12 der Zylinder-Kolben-Einheit 10 ist gegliedert in einen Einpressbereich 60 und in einen den Einpressbereich umgebenden Anpressbereich 80. Der Einpressbereich 60 grenzt an die Mündung 29 des Durchbruchs 25 an. Seine Stirnfläche 61 steht in der Injektionsrichtung 2 der Zylinder-Kolben-Einheit 10 im Ausführungsbeispiel um 1,5 Millimeter über die Stirnfläche 81 des Anpressbereichs 80 über. Dieser Versatz entspricht dem siebeneinhalbfachen Durchmesser der Mündung 29. Es ist auch denkbar, den Versatz geringer auszuführen, beispielsweise mit dem fünffachen Mündungsdurchmesser.

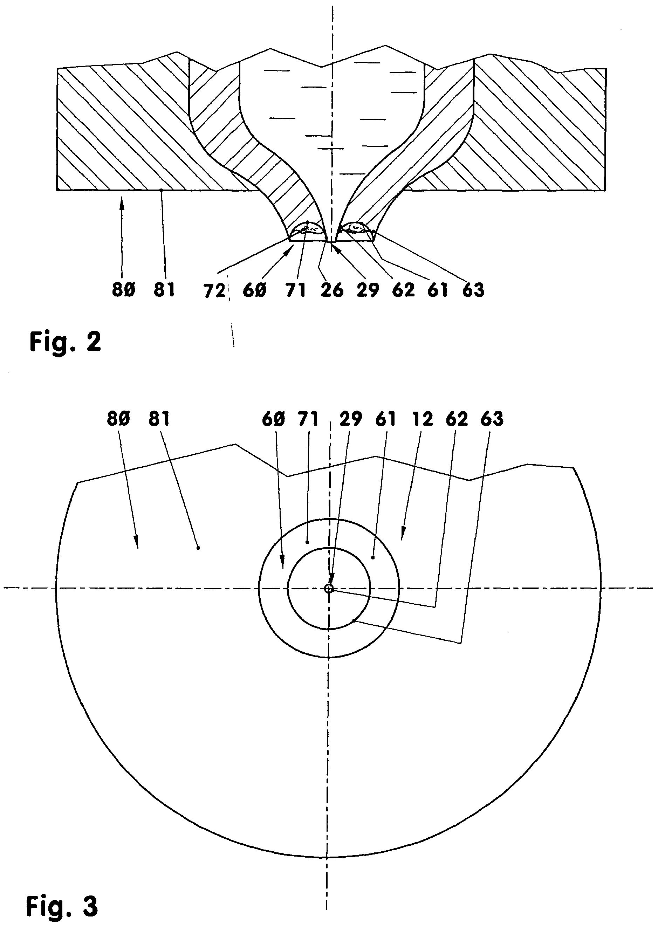

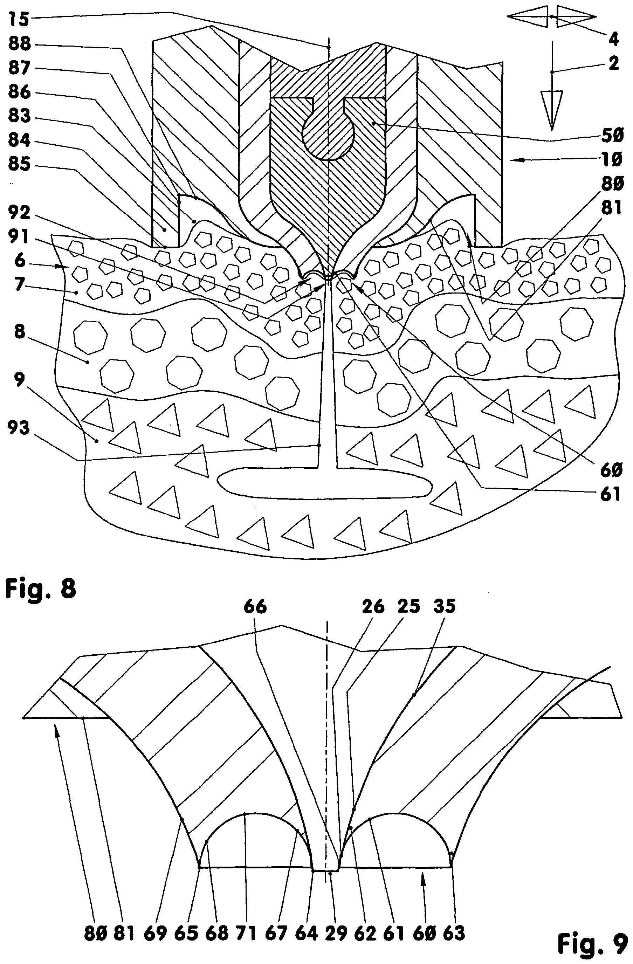

Der Einpressbereich 60 hat einen Außendurchmesser von z.B. zwei Millimetern. Er hat zwei, in der Injektionsrichtung 2 weisende, beispielsweise koaxial zueinander angeordnete Stege 62, 63, vgl. die Figuren 2 und 9. Beide Stege 62, 63 haben in dieser Richtung eine z.B. spitze Kante 64, 65. Diese Kanten 64, 65 spannen im Ausführungsbeispiel eine gedachte Kegelmantelfläche auf, deren Spitzenwinkel 175 Grad beträgt. Der Spitzenwinkel der Kegelmantelfläche kann zwischen 165 Grad und 180 Grad liegen, wobei die Kegelspitze außerhalb der Zylinder-Kolben-Einheit 10 liegt. Es ist auch denkbar, dass die beiden Stegkanten 64, 65 eine gemeinsame Ebene aufspannen. Diese Ebene liegt dann z.B. normal zur Mittelachse 15 der Zylinder-Kolben-Einheit 10.

Der innere Steg 62 umgreift die Mündung 29. Seine Innenflanke 66 bildet einen Teil der Wandung des Durchbruchs 25. Die konkav ausgebildete Außenflanke 67 schließt in der Kante 64 mit der Innenflanke 66 einen Winkel von 12 Grad ein.

Der äußere Steg 63 ist im Ausführungsbeispiel mittels zweier konkaver Flanken 68, 69 begrenzt. Der in der Kante 65 eingeschlossene Winkel beträgt beispielsweise 36 Grad.

Die von den Stegflanken 66, 67; 68, 69 in den Kanten 64, 65 eingeschlossene Winkel können bis zu 45 Grad betragen. Gegebenenfalls kann der einzelne Steg 62, 63 statt der scharfen Kante 64, 65 eine Ringfläche aufweisen, die z.B. eine Breite von 0,1 Millimeter hat. In diesem Fall schließen die an die Ringfläche angrenzenden Tangentialebenen der Stegflanken 66, 67; 68, 69 den genannten Winkel ein. Auch kann eine oder können beide Stegkanten 64, 65 als Halbtorus ausgebildet sein.

Die beiden Stege 62, 63 begrenzen eine umlaufende Rinne 71. Diese Ausnehmung 71 hat im Ausführungsbeispiel einen konstanten, halbkreisförmigen Querschnitt. Die Tiefe der Ausnehmung 71 beträgt z.B. 0,55 Millimeter. Beispielweise beträgt die Querschnittsfläche 0,48 Quadratmillimeter, das sind z.B. 15 % der in eine Normalenebene zur Mittelachse 15 der Zylinder-Kolben-Einheit 10 projizierten Stirnfläche des Einpressbereichs 60. Der Querschnitt der Ausnehmung 71 kann auch halboval, halbelliptisch, V-förmig, rechteckig etc. sein. Auch ein unsymmetrischer Querschnitt ist denkbar.

Die parallel zur Mittelachse 15 gemessene Tiefe der Ausnehmung 71 beträgt zwischen einem Viertel und drei Vierteln ihrer Ausdehnung in radialer Richtung 4 in einer Normalenebene zur Mittelachse 15. Sie kann entlang der Erzeugenden der Ausnehmung 71 - dies ist beispielsweise die Symmetrielinie der Ausnehmung 71 - variieren.

Der Anpressbereich 80 umfasst hier eine ebene Fläche 81. Sie liegt in einer Normalenebene zur Mittelachse 15 der Zylinder-Kolben-Einheit 10. Diese Fläche ist z.B. 66 Mal so groß wie die auf eine Normalenebene zur Mittelachse 15 projizierte Stirnfläche 61 des Einpressbereichs 60. Das Verhältnis der beiden Flächen kann auch kleiner sein. Die in eine Normalenebene zur Mittelachse 15 projizierte Stirnfläche 81 des Anpressbereichs 80 ist jedoch größer als die in diese Ebene projizierte Fläche 61 des Einpressbereichs 60.

In der Figur 1 ist um die Zylinder-Kolben-Einheit 10 herum eine Schiebehülse 13 angeordnet. Auf dieser sitzt eine Verschlusskappe 40. Diese besteht aus einem z.B. elastisch verformbaren Haltering 41 und einer in diesem aufgespannten Membran 42. Die Membran 42 liegt beispielsweise elastisch gespannt an der Außenkante der Zylinder-Kolben-Einheit 10 sowie an beiden Stegen 62, 63 an. Mit dieser Drei-LinienAuflage gewährleistet sie die Sterilität des Einpressbereichs 60 und des Anpressbereichs 80. Außerdem wirkt die Membran 42 als Überdruckventil, wenn z.B. vor dem Einsatz des Injektors Luft aus der Kammer gepresst wird.

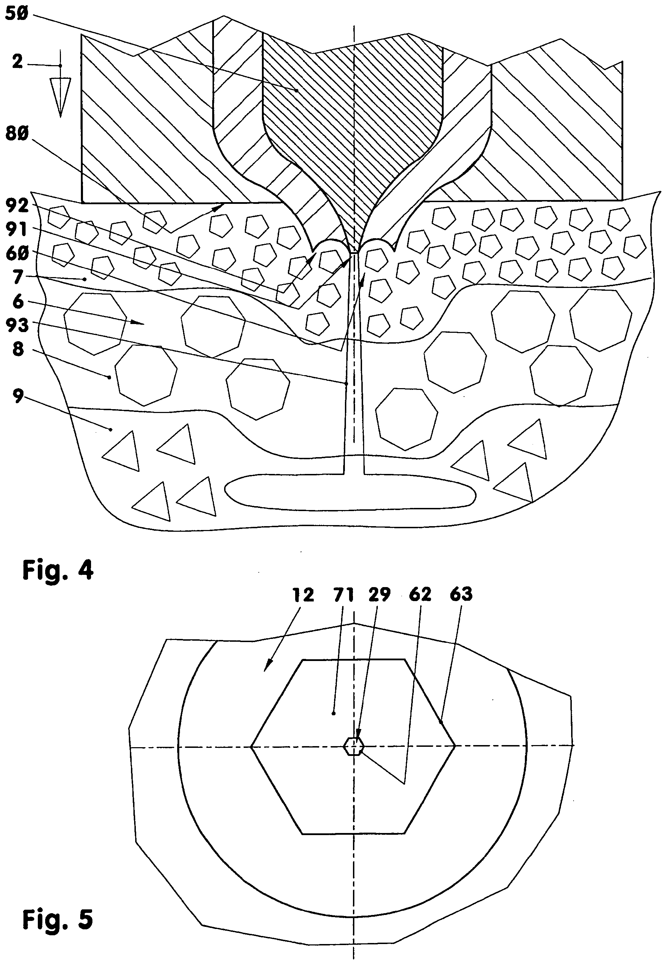

Zur Anwendung des Injektors, z.B. eines Einmalinjektors, wird - nach dem Füllen der Injektionslösung und dem Verdrängen der Luft aus dem Verdrängungsraum 30 - die Verschlusskappe 40 abgezogen. Anschließend wird der Injektor mit der außenliegenden Stirnseite 12 der Zylinder-Kolben-Einheit 10 auf die Haut 6 des Patienten aufgesetzt, vgl. Figur 4. Beim Aufsetzen berühren zunächst die Stege 62, 63 die Haut 6. Sie drücken die äußere, festere Hautschicht 7 ein und verformen diese elastisch. Gleichzeitig wird die lockerere Unterhaut 8 sowie das darunterliegende Unterhaut-Fettgewebe 9 verformt. Außerhalb des Einpressbereichs 60 drückt der Anpressbereich 80 auf die Haut 6. Hiermit wird die Haut festgehalten, so dass die durch den Einpressbereich 60 verursachte Verformung parallel zur Oberfläche orientierte Zugspannungen in der Haut 6 verursacht.

Bei der Verformung wird die Haut 6 in dem von dem inneren Steg 62 umschlossenen Bereich 91 gedehnt. Das festere Hautgewebe 7 wird gespannt. In dem Bereich 92, der von den beiden Stegen 62, 63 eingeschlossen ist, wird die Haut nicht eingedrückt. Die Haut 6 legt sich wie ein O-Ring z.B. formschlüssig in die Ausnehmung 71. Sie dichtet hierdurch den von dem inneren Steg 62 umgebenen Bereich ab, so dass die Injektionsflüssigkeit nicht entlang der Hautoberfläche austreten kann. Ein "wet shot" wird damit wirksam verhindert. Gleichzeitig verhindern die be- und entlasteten Bereiche 91, 92 der Haut 6 ein Verrutschen des Injektors.

Noch vorteilhafter ist es, wenn die Ausnehmung 71 mit Kleber 72 wie bei einem Heftpflaster oder Transdermalen Therapeutischen System (TTS) versehen ist. Dazu wird die Haut 6 direkt um die Mündung 29 der Düsenbohrung 26 mit hoher Klebkraft entgegen der Richtung des Injektionsstrahls festgehalten. Das hat zur Folge, dass man die Geschwindigkeit des Injektionsstrahls erheblich reduzieren kann ohne Gefahr zu laufen, dass ein "wet shot" auftritt. Auf diese Weise lässt sich de Energiespeicher reduzieren und alle Druck oder Kraft belasteten Teil kleiner dimensionieren.

Der eingesetzte Kleber/Klebstoff 72 ist beispielsweise ein Polyvinylether, ein Sythesekautschuk, Chlorbutadien-Elastomer, etc. Auch Mehrkomponenten-Klebstoffe sind einsetzbar. Das Abbinden erfolgt durch partielle Kristallisation nach dem Verdampfen des Lösemittels. Der Elastizitätsmodul des Klebstoffs entspricht beispielsweise dem Elastizitätsmodul der Stirnseite 12 der Zylinder-Kolben-Einheit 10. Der Klebstoff 72 kann auch ein Klebstoff 72 sein, der für Heftpflaster einsetzbar ist, z.B. Collemplastrum adhaesivum DAB 6. Beispielsweise beim Einsatz mit fettiger oder schwitzender Haut kann auch ein z.B. stärker klebender Silikonkleber eingesetzt werden.

Nach dem Auslösen des Injektors wird der Kolben 50 der Zylinder-Kolben-Einheit 10 nach vorne in Richtung der Mündung 29 geschoben. Die hierbei aus dem Verdrängungsraum 30 verdrängte Injektionsflüssigkeit wird bei einer subkutanen Injektion durch die festere, obere Hautschicht 7 und die Unterhaut 8 in das Unterhaut-Fettgewebe 9 verdrängt. Die gespannte, festere Hautschicht 7 verursacht hier nur einen geringeren Widerstand. Somit ist für die Injektion nur ein geringerer Druck erforderlich als bei ungespannter Haut. Der sich bei der Injektion bildende Hautkanal 93 schließt sich sicher und verhindert so ein Rückströmen der Injektionsflüssigkeit. Damit können technisch bedingte Unterdosierungen beim Patienten ausgeschlossen werden.

Die Figur 5 zeigt eine Ansicht der Stirnseite 12 einer Zylinder-Kolben-Einheit 10. Die Ringstege 62, 63 sind beispielsweise in Form eines Sechsecks angeordnet. Die zwischen den Ringstegen 62, 63 angeordnete Ausnehmung 71 hat in dieser Darstellung die Gestalt eines sechseckigen Rings. Der Querschnitt der Ausnehmung 71 entspricht beispielsweise dem Querschnitt der in den Figuren 1 - 4 dargestellten Ausnehmung 71. Die Stege 62, 63 können auch in Form eines Dreiecks, Vierecks, oder eines Polygonzuges angeordnet sein. Es ist denkbar, beide Stege 62, 63 in unterschiedlicher Gestalt auszuführen.

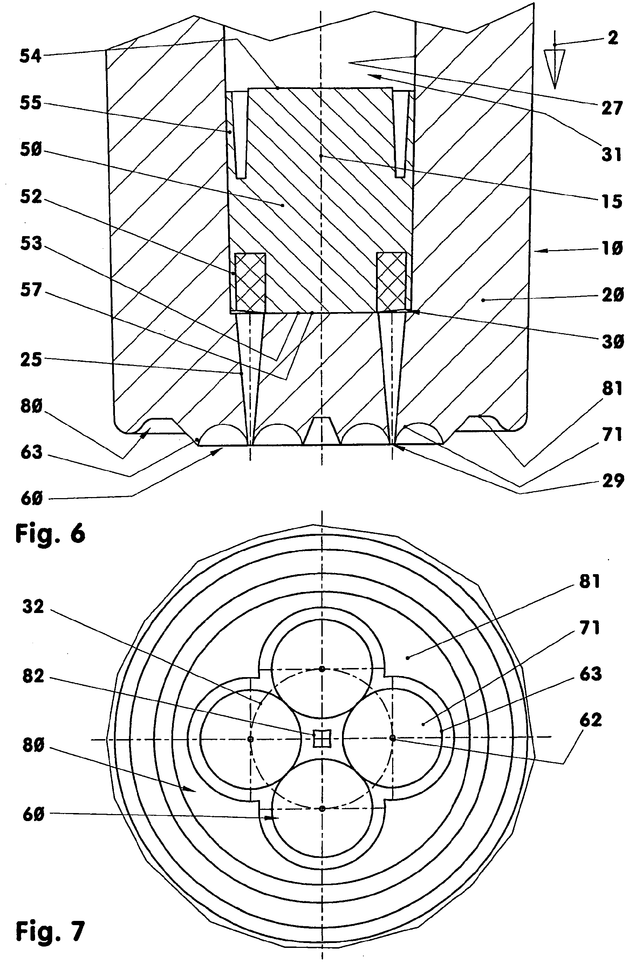

In den Figuren 6 und 7 ist eine weitere Zylinder-Kolben-Einheit 10 in einer Schnittdarstellung und in einer Ansicht entgegen der Injektionsrichtung 2 dargestellt. Der in diesen Figuren dargestellte Zylinder 20 ist beispielsweise aus einem EinKomponenten-Werkstoff hergestellt. Die Zylinder-Kolben-Einheit 10 weist z.B. vier den Zylinderinnenraum 31 mit der Umgebung 1 verbindende Durchbrüche 25 auf. Diese Durchbrüche 25 liegen auf einem gemeinsamen Teilkreis 32. Im Ausführungsbeispiel ist der Querschnitt aller Durchbrüche 25 identisch. Die einzelnen Durchbrüche 25 können jedoch auch unterschiedliche Querschnitte aufweisen.

Jeder Durchbruch 25 hat eine Mündung 29, die von einem Einpressbereich 60 umgeben ist. Der einzelne Einpressbereich 60 ist so aufgebaut wie im Zusammenhang mit den Figuren 1 - 4 beschrieben. Beispielsweise tangieren sich die äußeren Stege 63 der einzelnen Einpressbereiche 60. Ein von allen Einpressbereichen 60 umschlossener zentraler Bereich 82 liegt in einer Ebene mit dem die Einpressbereiche 60 umgebenden Anpressbereich 80. Die normal zur Mittelachse 15 angeordnete Fläche 81 des Anpressbereichs 80 beträgt in diesem Ausführungsbeispiel das Doppelte der Summe aller in eine Normalenebene zur Mittelachse 15 projizierten Stirnflächen 61 der Einpressbereiche 60. Die Projektion der Stirnfläche 81 des Anpressbereichs 80 ist damit größer als die Summe der Projektionen der Stirnflächen 61 aller Einpressbereiche 60.

Der Kolben 50 der Zylinder-Kolben-Einheit 10 weist an seinen beiden Enden 53, 54 abstehende, vorgespannte Dichtmanschetten 52, 55 auf. Diese werden beim Verschieben des Kolbens 50 an die Zylinderinnenwandung 27 angepresst. Beispielsweise ist die dem Verdrängungsraum 30 zugewandte Kolbenstirnseite 57 als ebene Fläche ausgeführt.

Anstatt der gezeigten vier Durchbrüche 25 ist auch eine Ausführung mit zwei, drei, fünf oder mehr Durchbrüchen denkbar. Einer der Durchbrüche 25 kann bei einer derartigen Ausführungsform zentral angeordnet sein.

Beim Aufsetzen eines Injektors mit einer derartigen Zylinder-Kolben-Einheit 10 wird die Haut des Patienten im Bereich jedes einzelnen Einpressbereichs 60 so verformt, wie im Zusammenhang mit dem ersten Ausführungsbeispiel beschrieben. Die Haut legt sich in die rinnenförmigen Ausnehmungen 71, so dass die Umgebung jeder einzelnen Mündung 29 abgedichtet ist.

Der Anpressbereich 80 verhindert ein Verrutschen der oberen Hautschicht 7, so dass diese mittels des Einpressbereichs 60 für die Injektion gespannt wird. Auch in diesem Ausführungsbeispiel ist damit eine druckarme, sichere Injektion möglich mit dem Vorteil, dass Blasenbildung vermieden wird, da das Gesamtvolumen auf mehrere Teilvolumina verteilt wird.

In der Figur 8 ist eine Zylinder-Kolben-Einheit 10 dargestellt, deren Hauptabmessungen den Hauptabmessungen der in den Figuren 1 - 4 dargestellten Zylinder-Kolben-Einheiten 10 entspricht. Auch der Aufbau des Einpressbereichs 60 entspricht dem dort beschriebenen Aufbau.

Der Anpressbereich 80 weist eine umlaufende Rinne 83 auf. Diese ist in radialer Richtung 4 nach außen hin mittels eines Niederhalters 84 begrenzt. Die Größe der Stirnfläche 85 des Niederhalters 84 entspricht dem zehnfachen der auf eine Normalenebene zur Mittelachse 15 der Zylinder-Kolben-Einheit 10 projizierten Stirnfläche 61 des Einpressbereichs 60.

Die an den Niederhalter 84 angrenzende Flanke 86 der beispielsweise annähernd V-förmigen Rinne 83 ist z.B. normal zur Stirnfläche 85 des Niederhalters 84 ausgerichtet. Die Tiefe der Rinne 83 entspricht im Ausführungsbeispiel dem Durchmesser des Einpressbereichs 60.

Die an den Nutgrund 87 angrenzende Innenflanke 88 ist konkav ausgebildet und geht zur Stirnfläche 81 hin in eine konvex gewölbte Fläche über.

Beim Aufsetzen der Zylinder-Kolben-Einheit 10 auf die Haut 6 wird diese, wie im Zusammenhang mit dem ersten Ausführungsbeispiel beschrieben, in dem vom Einpressbereich 60 belasteten Abschnitt 91 gespannt. In dem entgegen der Injektionsrichtung 2 versetzten Anpressbereich 80 wird die Haut mittels des Niederhalters 84 an einem Verrutschen gehindert. Im Bereich der Rinne 83 schmiegt sich die Haut 6 an die Kontur der Rinne 83 an und bildet so ein eine zweite, z.B. formschlüssige Abdichtung des vom inneren Steg 62 begrenzten Bereichs, so dass die Injektionsflüssigkeit in den Hautkanal 93 gezwungen wird. Gleichzeitig verhindert die im Bereich der Rinne 83 geringer belastete Haut 6 ein Verrutschen des Injektors.

Bei der Injektion kann die Injektionsflüssigkeit somit sicher in das Unterhaut-Fettgewebe 9 gelangen.

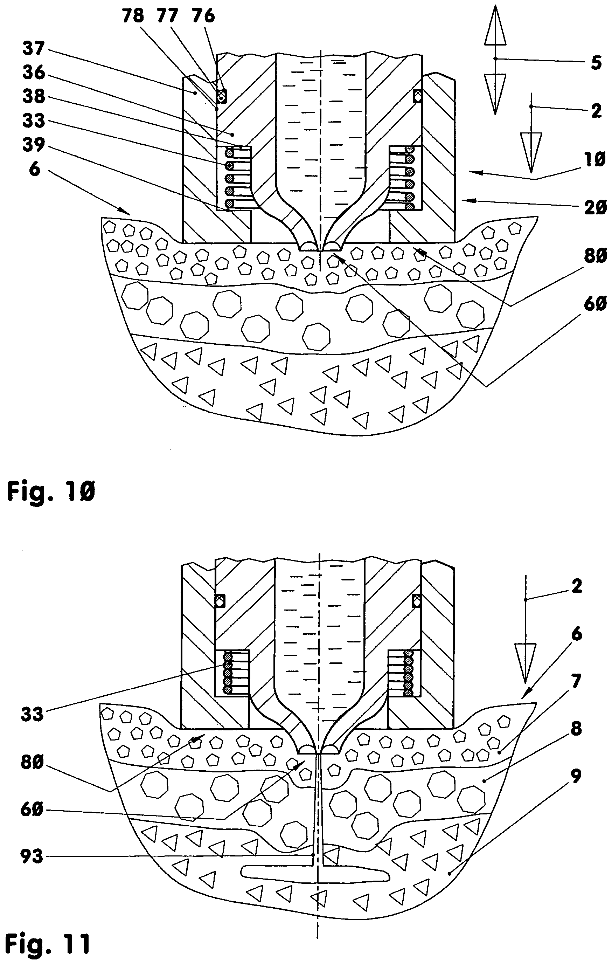

In den Figuren 10 und 11 ist eine weitere Zylinder-Kolben-Einheit 10 dargestellt. Der Zylinder 20 ist zweiteilig aufgebaut und hat einen inneren 36 und einen zu diesem koaxialen äußeren Teil 37. Der innere Teil 36 ist relativ zum äußeren Teil 37 in axialer Richtung 5 verschiebbar. Zwischen einer Stützfläche 38 des inneren Teils 36 und einer Anlagefläche 39 des äußeren Zylinderteils 37 ist eine Feder 33, z.B. eine Druckfeder 33 angeordnet. Ein in einer Ringnut 76 des Innenzylinders 36 eingesetzter O-Ring 77 verhindert das Eindringen von Keimen durch den Spalt 78 zwischen dem Innen- 36 und Außenzylinder 37 und dichtet so den Anpressbereich 80 steril ab.

Die Figur 10 zeigt die Zylinder-Kolben-Einheit 10 beim Aufsetzen auf die Haut des Patienten. Der Anpressbereich 80 des Außenzylinders 37 liegt an der Haut 6 an. Der Einpressbereich 60 des Innenzylinders 36 ist geringfügig in die Haut 6 eingedrückt. Er ist in dieser Ansicht in der Injektionsrichtung 2 z.B. um 0,5 Millimeter versetzt zum Anpressbereich 80. Der Einpressbereich 60 kann jedoch vor der Injektion auch entgegen der Injektionsrichtung 2 zum Anpressbereich 80 versetzt sein. Die Druckfeder 33 ist entlastet. Beispielsweise ist ein Auslösen des Injektors in diesem Zustand nicht möglich.

Beim weiteren Anpressen des Injektors wird der Innenzylinder 36 relativ zum Außenzylinder 37 in der Injektionsrichtung 2 verschoben, vgl. Figur 11. Die Feder 33 wird komprimiert. Hierdurch wird der Druck auf den Anpressbereich 80 erhöht. Gleichzeitig spannt der Einpressbereich 60 mit zunehmendem Hub die Haut 6. Der Einpressbereich 60 ist in der Darstellung der Figur 11 in der Injektionsrichtung 2 z.B. um 1,5 Millimeter gegenüber dem Anpressbereich 80 versetzt. Nun wird die Injektion freigegeben und ausgelöst. Die Injektionsflüssigkeit schießt unter Bildung eines Hautkanals 93 durch die Hautschichten 7, 8 in das Fettgewebe 9.

Auch Kombinationen der beschriebenen Ausführungsbeispiele sind denkbar.

### Bezugszeichenliste:

- 1: Umgebung
- 2: Injektionsrichtung
- 3: Präparat
- 4: radiale Richtung
- 5: axiale Richtung
- 6: Haut
- 7: feste Oberhaut
- 8: Unterhaut
- 9: Fettgewebe

- 10: Zylinder-Kolben-Einheit
- 11: Gewinde
- 12: Stirnseite von (10)
- 13: Schiebehülse

- 15: Mittellinie, Mittelachse
- 16: U-förmiges Profilteil, Sterilitätsverschluss
- 17: Öffnung von (17)
- 18: Dichtring
- 19: Steg

- 20: Zylinder
- 21: vorderes Ende
- 22: Zylinderwand
- 23: Innenwandschicht
- 24: Außenwandschicht
- 25: Durchbruch
- 26: Austrittselement, Düsenbohrung
- 27: Zylinderinnenwandung
- 28: Querschnittserweiterung
- 29: Mündung
- 30: Verdrängungsraum
- 31: Zylinderinnenraum
- 32: Teilkreis
- 33: Feder, Druckfeder
- 34: Zylinderboden
- 35: Ausströmtrichter
- 36: innerer Teil, Innenzylinder
- 37: äußerer Teil, Außenzylinder
- 38: Stützfläche
- 39: Anlagefläche
- 40: Verschlusskappe
- 41: Haltering
- 42: Membran
- 50: Kolben
- 52: Dichtlippe, Dichtmanschette
- 53: Ende von (50)
- 54: Ende von (50)
- 55: Dichtmanschette
- 56: Kolbenstange
- 57: Kolbenstirnseite

- 60: Einpressbereich
- 61: Stirnfläche
- 62: innerer Steg
- 63: äußerer Steg
- 64: Kante
- 65: Kante
- 66: Innenflanke
- 67: Außenflanke
- 68: Innenflanke
- 69: Außenflanke

- 71: Ausnehmung, Rinne
- 72: Kleber, Haftkleber
- 76: Ringnut
- 77: Dichtungsring, O-Ring
- 78: Spalt

- 80: Anpressbereich
- 81: Stirnfläche
- 82: zentraler Bereich
- 83: Rinne, Ausnehmung
- 84: Niederhalter
- 85: Stirnfläche von (84)
- 86: Flanke
- 87: Nutgrund
- 88: Innenflanke

- 91: Bereich von (6)
- 92: Bereich von (6)
- 93: Hautkanal

## Patentansprüche

1. Zylinder-Kolben-Einheit (10) eines Einmalinjektors mit mindestens einem, den Zylinderinnenraum (31) mit einer stirnseitigen Mündung (29) verbindenden Durchbruch (25),
- wobei eine Stirnseite (12) der Zylinder-Kolben-Einheit (10) einen die Mündung (29) umgreifenden Einpressbereich (60) und einen den Einpressbereich (60) umgebenden Anpressbereich (80) aufweist,
- wobei der Einpressbereich (60) einen inneren, die Mündung (29) begrenzenden Steg (62) und einen äußeren Steg (63) umfasst,
- wobei die Stege (62, 63) eine den inneren Steg (62) umgebende Ausnehmung (71) begrenzen, deren parallel zur Mittelachse (15) der Zylinder-Kolben-Einheit (10) gemessene Tiefe mindestens ein Viertel ihrer Breite in einer normal hierzu orientierten Ebene beträgt und
- wobei die in eine Normalenebene zur Mittelachse (15) projizierte Fläche (81) des Anpressbereichs (80) mindestens so groß ist wie die in dieselbe Ebene projizierte Fläche (61) des Einpressbereichs (60), **dadurch gekennzeichnet,**
- **dass** der innere Steg (62) eine konkav ausgebildete Außenflanke (67) und eine Innenflanke (66) aufweist, die in einer Kante (64) einen Winkel von bis zu 45 Grad einschließen,
- **dass** der äußere Steg (63) mittels zweier konkaver Flanken (68, 69) begrenzt ist, die in einer Kante (65) einen Winkel von bis zu 45 Grad einschließen,
- **dass** die Kanten (64, 65) entweder in einer Ebene normal zur Mittelachse (15) liegen oder eine kegelförmige Fläche aufspannen, deren Spitzenwinkel zwischen 165 Grad und 180 Grad beträgt und deren Kegelspitze außerhalb der Zylinder-Kolben-Einheit (10) liegt und
- **dass** der Anpressbereich (80) zumindest während einer Injektion entgegen der Injektionsrichtung (2) zu der Stirnfläche (61) des Einpressbereichs (60) versetzt ist.

2. Zylinder-Kolben-Einheit (10) nach Anspruch 1,
**dadurch gekennzeichnet,**
**dass** der Querschnitt der Ausnehmung (71) entlang ihrer Erzeugenden konstant ist.

3. Zylinder-Kolben-Einheit (10) nach Anspruch 1,
**dadurch gekennzeichnet,**
**dass** die Ausnehmung (71) klebend ist.

4. Zylinder-Kolben-Einheit (10) nach Anspruch 1,
**dadurch gekennzeichnet,**
**dass** der Anpressbereich (80) eine Fläche umfasst, die in einer Normalenebene zur Mittelachse (15) der Zylinder-Kolben-Einheit (10) liegt.

5. Zylinder-Kolben-Einheit (10) nach Anspruch 1,
**dadurch gekennzeichnet,**
**dass** der Anpressbereich (80) eine Ausnehmung (83) aufweist.

6. Zylinder-Kolben-Einheit (10) nach Anspruch 5,
**dadurch gekennzeichnet,**
**dass** die Ausnehmung (83) koaxial zur Mittelachse (15) der Zylinder-Kolben-Einheit (10) ist.

7. Zylinder-Kolben-Einheit (10) nach Anspruch 1,
**dadurch gekennzeichnet,**
**dass** sie mindestens zwei Durchbrüche (25) umfasst.

8. Zylinder-Kolben-Einheit (10) nach Anspruch 1,
**dadurch gekennzeichnet,**
**dass** der Zylinder (20) der Zylinder-Kolben-Einheit (10) einen Innenzylinder (36) und eine diesen umgebenden Außenzylinder (37) umfasst.

9. Zylinder-Kolben-Einheit (10) nach Anspruch 8,
**dadurch gekennzeichnet,**
**dass** der Innenzylinder (36) gegenüber dem Außenzylinder (37) in der Injektionsrichtung (2) unter Belastung einer Feder (33) verschiebbar ist.

## Claims

1. Cylinder-piston unit (10) in a disposable injector, said unit having at least one opening (25) communicating the interior (31) of the cylinder with an aperture (29) in the end face thereof, wherein
- an end face (12) of the cylinder-piston unit (10) has a pressing-in portion (60) surrounding the aperture (29) and a pressing-on portion (80) surrounding the pressing-in portion (60),
- the pressing-in portion (60) has an inner ridge (62) delimiting the aperture (29) and an outer ridge (63),
- the ridges (62, 63) delimit a recess (71) surrounding the inner ridge (62), said recess having in parallel to the central axis (15) of the cylinder-piston unit (10) a depth amounting to at least one quarter of its width in a plane oriented normal thereto, and
- the area (81) of the pressing-on portion (80) projected in a plane normal to the central axis (15) is at least equal in surface area to the area (61) of the pressing-in portion (60) projected in the same plane, **characterized in that**
- the inner ridge (62) has a concavely shaped outer flank (67) and an inner flank (66) which flanks include an angle of up to 45 degrees at an edge (64),
- the outer ridge (63) is delimited by a pair of concave flanks (68, 69), said flanks including an angle of up to 45 degrees at an edge (65),
- said edges (64, 65) either lie in a plane normal to the central axis (15) or define a conical surface of which the apex angle amounts to between 165 degrees and 180 degrees and of which the apex lies outside the cylinder-piston unit (10), and
- the pressing-on portion (80) is offset from the end face (61) of the pressing-in portion (60) against the direction of injection (2) at least during an injection.

2. Cylinder-piston unit (10) as claimed in claim 1, **characterized by** the cross section of recess (71) being constant along the generatrix thereof.

3. Cylinder-piston unit (10) as claimed in claim 1, **characterized by** the recess (71) having adhesive properties.

4. Cylinder-piston unit (10) as claimed in claim 1, **characterized by** the pressing-on portion (80) comprising an area defined in a plane normal to the central axis (15) of the cylinder piston unit (10).

5. Cylinder-piston unit (10) as claimed in claim 1, **characterized by** the pressing-on portion (80) having a recess (83) therein.

6. Cylinder-piston unit (10) as claimed in claim 5, **characterized by** the recess (83) being coaxial with the central axis (15) of the cylinder-piston unit (10).

7. Cylinder-piston unit (10) as claimed in claim 1, **characterized by** having at least two openings (25) therethrough.

8. Cylinder-piston unit (10) as claimed in claim 1, **characterized in that** the cylinder (20) of the cylinder-piston unit (10) comprises an inner cylinder (36) and an outer cylinder (37) surrounding said inner cylinder.

9. Cylinder-piston unit (10) as claimed in claim 8, **characterized in that** the inner cylinder (36) is adapted to be slidingly displaced relative to the outer cylinder (37) in the direction of injection (2) under a bias exerted by a spring (33).

## Revendications

1. Unité cylindre-piston (10) d'un injecteur à usage unique pourvu d'au moins un passage (25) reliant l'espace intérieur du cylindre (31) à une embouchure (29) du côté frontal,
- un côté frontal (12) de l'unité cylindre-piston (10) présentant une zone d'enfoncement (60) enveloppant l'embouchure (29) et une zone de pression (80) entourant la zone d'enfoncement (60),
- la zone d'enfoncement (60) comprenant une nervure intérieure (62) délimitant l'embouchure (29) et une nervure extérieure (63),
- les nervures (62, 63) limitant un évidement (71) entourant la nervure intérieure (62) dont la profondeur mesurée parallèlement à l'axe central (15) de l'unité cylindre-piston (10) représente au moins un quart de sa largeur dans un plan orienté normalement à celle-ci et
- la surface (81) de la zone de pression (80) projetée dans un plan normal à l'axe central (15) étant au moins aussi grande que la surface (61) de la zone d'enfoncement (60) projetée dans ce même plan, **caractérisé en ce**
- **que** la nervure intérieure (62) présente un flanc extérieur concave (67) et un flanc intérieur (66) qui forment une arête (64) où elles renferment un angle jusqu'à 45 degrés,
- **que** la nervure extérieure (63) est délimitée par deux surfaces concaves (68, 69) qui forment une arête (65) où elles renferment un angle jusqu'à 45 degrés,
- **que** les arêtes (64, 65) se situent dans un plan perpendiculaire à l'axe central (15) ou qu'elles définissent une surface conique dont l'angle de pointe est entre 165 et 180 degrés et dont la pointe conique se trouve à l'extérieur de l'unité cylindre-piston (10) et
- **que** la zone de pression (80) est décalée, au moins pendant une injection, par rapport à la face frontale (61) de la zone d'enfoncement (60) dans le sens opposé à la direction de l'injection (2).

2. Unité cylindre-piston (10) selon la revendication 1, **caractérisée en ce**
**que** la section de l'évidement (71) est constante le long de sa génératrice.

3. Unité cylindre-piston (10) selon la revendication 1, **caractérisée en ce**
**que** l'évidement (71) est adhésif.

4. Unité cylindre-piston (10) selon la revendication 1, **caractérisée en ce**
**que** la zone de pression (80) comprend une surface qui se trouve dans un plan normal à l'axe central (15) de l'unité cylindre-piston (10).

5. Unité cylindre-piston (10) selon la revendication 1, **caractérisée en ce**
**que** la zone de pression (80) présente un évidement (83).

6. Unité cylindre-piston (10) selon la revendication 5, **caractérisée en ce**
**que** l'évidement (83) est coaxial à l'axe central de l'unité cylindre piston (10).

7. Unité cylindre-piston (10) selon la revendication 1, **caractérisée en ce**
**qu'**elle comprend au moins deux passages (25).

8. Unité cylindre-piston (10) selon la revendication 1, **caractérisée en ce**
**que** le cylindre (20) de l'unité cylindre-piston (10) comprend un cylindre intérieur (36) et un cylindre extérieur (37) entourant ce dernier.

9. Unité cylindre-piston (10) selon la revendication 8, **caractérisée en ce**
**que** le cylindre intérieur (36) peut être déplacé par rapport au cylindre extérieur (37) dans la direction de l'injection (2) l'aide de la sollicitation d'un ressort (33).
